# EUROPEAN PATENT APPLICATION

(11) **EP 4 230 719 A1**
(43) Date of publication of application: **23.08.2023**
(21) Application number: 21879902.1
(22) Date of filing: 04.10.2021
(51) Int. Cl.: C12M 3/00, C12N 5/071, C12Q 1/02, G02B 21/26, G02B 21/34

(54) **CELL CULTURE VESSEL, OBSERVATION DEVICE, MICROSCOPE, CULTURE METHOD, AND OBSERVATION METHOD**

(30) Priority: 15.10.2020 JP 2020173993
(71) Applicant: RIKEN, Wako-shi, Saitama 351-0198 (JP)
(72) Inventor: HAGIWARA, Masaya, Wako-shi, Saitama 351-0198 (JP)
(74) Representative: Graf von Stosch Patentanwaltsgesellschaft mbH
(86) International application number: PCT/JP2021/036555
(87) International publication number: WO 2022/080162

(57) **Abstract**

A cell culture vessel of the present invention includes a plurality of compartments each having a corresponding three-dimensional shape and each configured to be able to retain therein an aqueous liquid by surface tension, or includes a plurality of compartments surrounded by a framework corresponding to sides of a polyhedral shape. Adjacent compartments communicate with each other.

## Description

### Technical Field

The present invention relates to a cell culture vessel, an observation apparatus, a microscope, a culture method, and an observation method.

### Background Art

Recent attention has been paid to pharmaceutical development with use of an organoid as a new in vitro experimental system. A complex biological system can be reproduced by means of an organoid, and a medicine with high therapeutic efficacy is expected to be developed.

In contrast, a technique for three-dimensionally culturing a tissue such as an organoid has not been established. In a common three-dimensional culture method, only one type of extracellular matrix is used. It is therefore impossible to control the cell distribution of a tissue. The extracellular matrix is diverse even in a single organ depending on a site. Positions of cells constituting an organ determine differentiation, development, and localization of the cells. Thus, three-dimensional disposition of cells and a somatic matrix is important, and various studies have been carried out.

For example, Non-patent Literature 1 discloses the possibility of reproducing various in vivo phenomena by perfusing various tissues in a microfluidic device. Furthermore, Non-patent Literature 2 discloses a three-dimensional culture method carried out with use of 3D bioprinting.

### Citation List

### [Non-patent Literature]

[Non-patent Literature 1]
   Nashimoto et. al., Integr. Biol., 9(6), p.506-518, 2017
[Non-patent Literature 2]
   Lee et al., Science, Vol.365, Issue 6452, p.482-487, 2019

### Summary of Invention

### Technical Problem

However, such conventional techniques as those disclosed above unfortunately require a special apparatus and a special technique. Furthermore, in the conventional techniques, condition setting is also unfortunately not easy. Thus, a three-dimensional culture method that does not require any special apparatus or technique and that anyone can easily carry out is desired to be developed.

An object of the present invention is to achieve, for example, a vessel for three-dimensional culture that does not require any special apparatus or technique and that anyone can easily carry out.

### Solution to Problem

The inventor of the present invention repeatedly carried out diligent study in order to attain the above object, and consequently found that it is possible to separate gels by changing compositions of an aqueous liquid forming a culture gel that embeds a cell or cell tissue and an aqueous liquid located around the culture gel, and using a difference in surface tension between these aqueous liquids. That is, the inventor of the present invention finally accomplished the present invention by finding that even a simple and easy operation allows organoid culture to be designed with a greatly increased degree of freedom.

In order to attain the object, the present invention includes the following aspects.
· A cell culture vessel including a plurality of compartments each having a corresponding three-dimensional shape and each configured to be able to retain therein an aqueous liquid by surface tension, wherein any adjacent compartments out of the plurality of compartments communicate with each other.
· A cell culture vessel including a plurality of compartments surrounded by a framework corresponding to sides of a polyhedron, the plurality of compartments each being configured to be able to retain therein an aqueous liquid by surface tension.
· A method of culturing a cell or cell tissue accommodated in a cell culture vessel that has an external form having a polyhedral shape, the cell culture vessel including a plurality of compartments each having a corresponding three-dimensional shape and each configured to be able to retain therein an aqueous liquid by surface tension, wherein any adjacent compartments out of the plurality of compartments communicate with each other, or including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other, the method including accommodating, in the plurality of compartments, a gel composition including a cell or cell tissue.
· A method of observing a cell or cell tissue accommodated in a cell culture vessel that has an external form having a polyhedral shape, the cell culture vessel including a plurality of compartments each having a corresponding three-dimensional shape and each configured to be able to retain therein an aqueous liquid by surface tension, wherein any adjacent compartments out of the plurality of compartments communicate with each other, or including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other, the cell culture vessel further including a shaft part which extends, from any of vertices of the polyhedral shape, outward in a direction that is not parallel to any of lines normal to faces of the polyhedral shape, the method including rotating the cell culture vessel on the shaft part.
· A method of observing a cell or cell tissue accommodated in a cell culture vessel that has an external form having a polyhedral shape, the cell culture vessel including a plurality of compartments each having a corresponding three-dimensional shape and each configured to be able to retain therein an aqueous liquid by surface tension, wherein any adjacent compartments out of the plurality of compartments communicate with each other, or including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other, a fixing tool for fixing the cell culture vessel including: a holding part which holds the cell culture vessel; and a shaft part which is connected to the holding part and which extends, from any of vertices of the polyhedral shape, outward in a direction that is not parallel to any of lines normal to faces of the polyhedral shape, the method including using the fixing tool to hold the cell culture vessel and rotating the cell culture vessel on the shaft part.

### Advantageous Effects of Invention

An aspect of the present invention makes it possible to achieve, for example, a vessel for three-dimensional culture that does not require any special apparatus or technique and that anyone can easily carry out.

### Brief Description of Drawings

Fig. 1 is a drawing schematically illustrating an example of a cell culture vessel of an aspect of the present invention.
Fig. 2 is an enlarged view of a part of the cell culture vessel illustrated in Fig. 1.
Fig. 3 is an enlarged view of a part of the cell culture vessel illustrated in Fig. 1.
Fig. 4 is a drawing illustrating an example of a cell culture vessel of an aspect of the present invention.
Fig. 5 is a drawing illustrating an example of a fixing tool of an observation apparatus of an aspect of the present invention.
Fig. 6 is a drawing illustrating a state in which the fixing tool illustrated in Fig. 5 is fitted on the cell culture vessel.
Fig. 7 is a perspective view illustrating a state in which an example of the observation apparatus of an aspect of the present invention is mounted on a microscope.
Fig. 8 is a drawing showing a result of Evaluation Example 1.
Fig. 9 is a drawing showing a result of Evaluation Example 2.
Fig. 10 is a drawing showing a result of Evaluation Example 3.
Fig. 11 is a drawing showing a result for day 0 of culture in Example 1.
Fig. 12 is a drawing showing a result for day 8 of culture in Example 1.
Fig. 13 is a drawing illustrating a framework (frame) of a cell culture vessel, the framework having a substantially quadrangular cross-section.
Fig. 14 is a drawing illustrating a frame of a cell culture vessel, the frame having a substantially L-shaped cross-section.
Fig. 15 is a drawing illustrating preparation of a cell culture vessel with use of a frame of a cell culture vessel, the frame having a substantially quadrangular cross-section.
Fig. 16 is a drawing illustrating preparation of a cell culture vessel with use of a frame of a cell culture vessel, the frame having a substantially quadrangular cross-section.
Fig. 17 is a drawing illustrating preparation of a cell culture vessel with use of a frame of a cell culture vessel, the frame having a substantially L-shaped cross-section.
Fig. 18 is a drawing illustrating a frame of a cell culture vessel, the frame having a substantially L-shaped cross-section.
Fig. 19 is a drawing illustrating an evaluation method in Evaluation Example 5.
Fig. 20 is a drawing showing a result of Evaluation Example 5.
Fig. 21 is a drawing related to a curve on an interface between hydrogel and air.
Fig. 22 is a drawing related to a curve on an interface between hydrogel and air.
Fig. 23 is a drawing illustrating a cell culture vessel prepared in Evaluation Example 6.
Fig. 24 is a drawing illustrating a cell culture vessel prepared in Evaluation Example 6.
Fig. 25 is a drawing illustrating a cell culture vessel prepared in Evaluation Example 6.
Fig. 26 is a drawing illustrating a cell culture vessel prepared in Evaluation Example 6.
Fig. 27 is a drawing illustrating a cell culture vessel prepared in Evaluation Example 6.
Fig. 28 is a drawing illustrating a cell culture vessel prepared in Evaluation Example 6.
Fig. 29 is a drawing schematically illustrating a cell culture vessel used in Example 2.
Fig. 30 is a drawing showing a result obtained after 96 hours of culture of NHBE in Example 2.
Fig. 31 is a drawing showing a result obtained after 92 hours of culture of a mouse endothelial cell in Example 2.

### Description of Embodiments

### [1. Cell culture vessel]

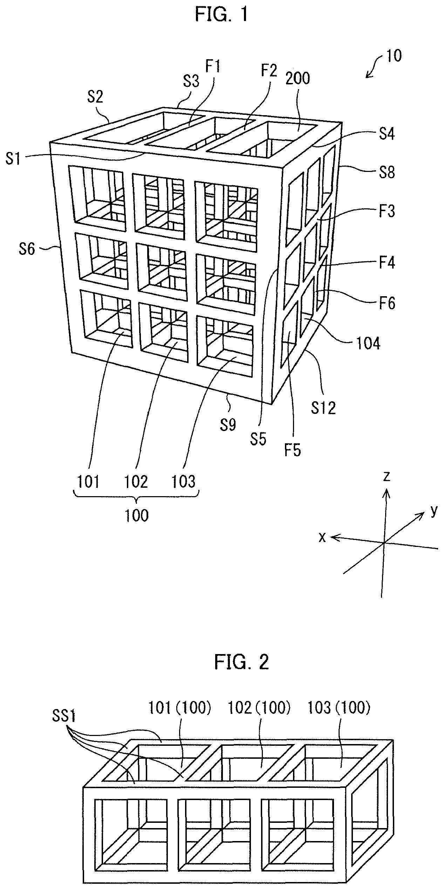

An example of a cell culture vessel in accordance with an aspect of the present invention will be described in detail with reference to Figs. 1 to 4.

A cell culture vessel 10 is a vessel for accommodating and culturing a cell or cell tissue therein. The cell or cell tissue is accommodated in the cell culture vessel 10 together with an aqueous liquid. The aqueous liquid herein refers to a liquid before gelation. The aqueous liquid will be described later.

The cell culture vessel 10 in accordance with an aspect of the present invention is configured to have, for example, an external form having a substantially cubic shape, as illustrated in Fig. 1. More specifically, the cell culture vessel 10 has a substantially cubic shape having 12 sides S1 to S12, as illustrated in Fig. 1. A height direction of the cell culture vessel 10 is referred to as a z direction, a direction parallel to S4 or S12 is referred to as a y direction, and a direction parallel to S1 or S9 is referred to as an x direction. Note, however, that the shape of the cell culture vessel 10 is not limited to a cubic shape. The cell culture vessel 10 may have another polyhedral shape such as a rectangular parallelepiped shape or a spherical shape. The cell culture vessel 10 preferably has a hexahedral shape (external form), and more preferably has a cubic shape (external form) from the viewpoint of, for example, ease of handling of the cell culture vessel 10. The size of the cell culture vessel 10 can be, for example, such that an outer diameter of the cell culture vessel 10 has an outer dimension of approximately 4 mm and an inner dimension of approximately 3 mm. Note, however, that the specific size of the cell culture vessel 10 does not limit the present embodiment. Note that the x direction, the y direction, and the z direction are not limited to those defined above and refer to three directions which extend from a single origin and in which vectors indicating the respective three directions are orthogonal to each other.

### (Outer framework 11 and frame parts F1 to F6 of vessel)

The cell culture vessel 10 includes an outer framework 11 thereof corresponding to sides of a polyhedral shape (cubic shape). The cell culture vessel 10 has an inner part that is divided into a plurality of compartments 100 and 200 by the outer framework 11 and frame parts F1 to F6. The frame parts F1 to F4 constitute a frame including (i) a square-shaped outer frame having four sides and (ii) a partition dividing an inner part of the outer frame crosswise into substantially nine equal parts. That is, the frame parts F1 to F4 constitute a frame in which the inner part of the outer frame is divided in the form of nine substantially square meshes. The frame parts F5 and F6 constitute a frame including (i) a U-shaped outer frame having three sides and (ii) a partition dividing an inner part of the outer frame crosswise into substantially nine equal parts. That is, the frame parts F5 and F6 constitute a U-shaped frame in which the inner part of the outer frame is divided in the form of nine substantially square meshes. Note that for example, the number, shape, and arrangement of the frame parts can be selected as appropriate in accordance with how to divide the inner part of the cell culture vessel 10.

In order to, for example, maintain surface tension of the aqueous liquid accommodated in the cell culture vessel 10, the cell culture vessel 10 preferably includes a frame part so that each face of the cell culture vessel 10, the each face having a polyhedral shape, is divided into a plurality of compartments, and more preferably includes a frame part so that the each face of the polyhedral shape is divided into a plurality of equal parts. In the example of the cell culture vessel 10 illustrated in Fig. 1, five faces are divided in the form of nine substantially square-shaped meshes, and one face is divided in the form of three substantially rectangular meshes.

A material of the outer framework 11 and the frame parts F1 to F6 may be, for example, a biocompatible resin such as polycarbonate. Examples of a resin include a resin for use in a 3D printer. The outer framework 11 and the frame parts F1 to F6 are preferably treated with a surface treatment agent, such as polydimethylsiloxane (PDMS) in order to increase surface tension of the aqueous liquid accommodated in the cell culture vessel 10. Furthermore, the outer framework 11 and the frame parts F1 to F6 are preferably transparent in terms of, for example, ease of observation of the cell or cell tissue.

### (Compartments 100 and 200)

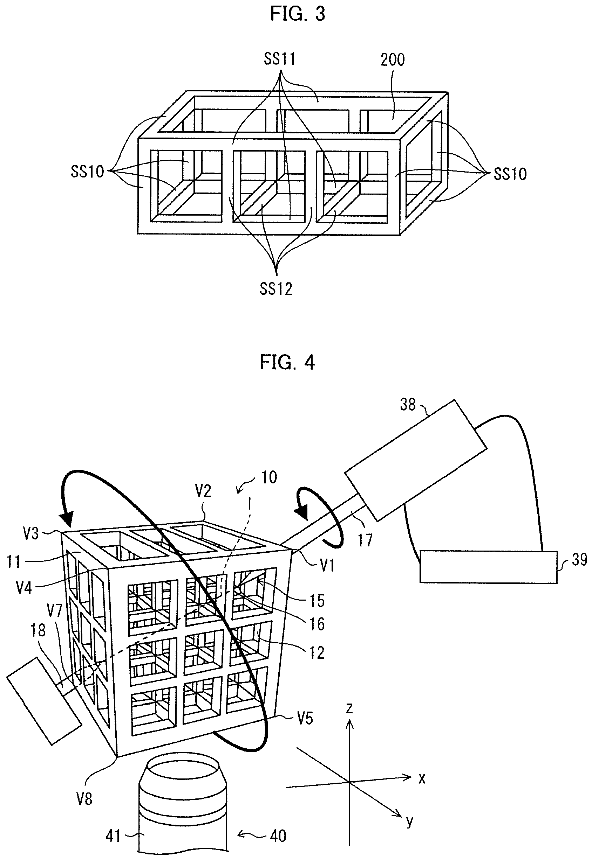

The cell culture vessel 10 includes a plurality of compartments 100 having a solid shape (three-dimensional shape) and a plurality of compartments 200 having a solid shape (three-dimensional shape). Fig. 2 is a drawing illustrating removed three adjacent compartments 100 (compartments 101 to 103). Fig. 3 is a drawing illustrating a removed single compartment 200. Each of the compartments 100 is a compartment surrounded by a framework SS1 corresponding to sides of a cube. Each of the compartments 200 is a compartment surrounded by frameworks SS10 and SS11 corresponding to sides of a rectangular parallelepiped. A compartment 200 that has rectangular side and bottom faces provided with an auxiliary framework SS12 is constituted by faces that are divided in the form of substantially square-shaped meshes, except for a top face thereof. Note that each of the compartments 100 and 200 consists of only a framework corresponding to sides of the three-dimensional shape, and a part corresponding to a face of the three-dimensional shape is open.

Each of the compartments 100 and 200 is able to retain therein the aqueous liquid by surface tension. This makes it possible to store the aqueous liquid in units of the compartments 100 and 200. That is, the aqueous liquid that has a corresponding different composition can also be stored in each of the compartments 100 and 200. In order to retain the aqueous liquid by surface tension in a compartment, a single compartment has a volume in a range of, for example, 0.001 mm³ to 10,000 mm³, preferably in a range of 1 mm³ to 500 mm³, and more preferably in a range of 8 mm³ to 300 mm³. In the case of a compartment having a cubic shape, such as a compartment 100, the framework SS1 corresponding to the sides of the cube has a length in a range of, for example, 0.1 mm to 100 mm, preferably in a range of 1 mm to 10 mm, and more preferably in a range of 1 mm to 5 mm. In a case where the side and bottom faces of a compartment having a larger volume, such as a compartment 200 are constituted by faces divided in the form of meshes, it is possible to retain the aqueous liquid in a larger amount by surface tension. Note that, in a case where the side and bottom faces are divided in the form of meshes having a quadrangular shape (e.g., substantially square shape), the quadrangular shape only needs to have sides having a length equivalent to the length of the framework SS1.

The compartments 100 are placed side by side in three directions, i.e., the x direction, the y direction, and the z direction, as illustrated in Fig. 1. In the cell culture vessel 10, a first layer in which nine compartments 100 are placed in an x-y direction without any gap and a second layer in which nine compartments 100 are placed in the x-y direction without any gap are stacked. In the cell culture vessel 10, a third layer in which three compartments 200 are placed in the x-y direction without any gap is further stacked on top of the second layer. Note that adjacent compartments (e.g., compartments 101 and 102) share a framework thereof in adjacent regions and communicate with each other. Note that the number of compartments provided is an example and is not particularly limited.

The compartments 100 and 200 are placed side by side preferably in the x direction and the y direction, the y direction and the z direction, or the x direction and the z direction, and more preferably in the x direction, the y direction, and the z direction, from the viewpoint of (i) maintenance of surface tension of the aqueous liquid accommodated in the cell culture vessel 10 and (ii) ease of design of three-dimensional disposition.

The shape of the compartments 100 and 200 is not limited to a hexahedral shape (cubic or rectangular parallelepiped shape). The compartments 100 and 200 may have another three-dimensional shape such as a polyhedral or spherical shape. Each of the compartments preferably has a cubic shape in order to, for example, maintain surface tension of the aqueous liquid. The compartments may have sizes identical to or different from each other. The compartments preferably have sizes identical to each other in order to, for example, maintain surface tension of the aqueous liquid.

As described above, the compartments 100 and 200 are each configured to be able to retain therein the aqueous liquid by surface tension. Furthermore, adjacent compartments (compartments 100, compartments 200, and compartments 100 and 200) communicate with each other. Such a configuration enables culture in a state in which a plurality of different cells and/or extracellular matrices are appropriately disposed (three-dimensionally disposed). Achievement of appropriate three-dimensional disposition makes it possible to, for example, form biological systems such as an angioplasty system and a multi-organ linkage system (e.g., a linkage between the liver and the bile duct).

### (Window part 12)

A window part 12 is surrounded by the 12 sides constituting the outer framework 11. That is, the window part 12 is constituted by six faces (i.e., a face constituted by V1, V2, V3, and V4, a face constituted by V1, V2, V6, and V5, a face constituted by V2, V3, V7, and V6, a face constituted by V3, V4, V8, and V7, a face constituted by V1, V4, V8, and V5, and a face constituted by V5, V6, V7, and V8) constituting the cubic shape illustrated in Fig. 1. A material of the window part 12 is preferably a material which is light-transmissive and which is permeable to a nutrient, a stimulating factor, and/or the like necessary to culture the cell or cell tissue (hereinafter also referred to as "sample") accommodated in the cell culture vessel 10. Specifically, the material of the window part 12 preferably contains, for example, agarose gel, polyacrylamide gel, sodium alginate, or collagen gel. In this manner, by making the window part 12 from such a nutrient-permeable material and immersing the cell culture vessel 10 in a liquid culture medium, a nutrient, a stimulating factor, and/or the like contained in the liquid culture medium are/is supplied, through the window part 12, to the cell or cell tissue accommodated in the cell culture vessel 10. The window part 21 may have a role of a wall part (described later).

Furthermore, by making the window part 12 from a light-transmissive material, it is possible to observe the cell or cell tissue accommodated inside the cell culture vessel 10, from directions of the plurality of faces constituting the cubic shape.

### (Shaft part 17)

As illustrated in Fig. 4, the cell culture vessel 10 may include a shaft part 17 which extends, from any of the vertices of the polyhedral shape, outward in a direction that is not parallel to any of lines normal to the faces of the polyhedral shape.

As an example, the shaft part 17 extends from one vertex V1 of the cell culture vessel 10, which has a cubic shape, in a direction along a straight line 1 connecting the vertex V1 and the vertex V7, which is located at a diagonal position with respect to the vertex V1 (see Fig. 1). Note that a bearing 18 may be provided at the vertex V7, which is located at a diagonal position with respect to the vertex V1.

Alternatively, the shaft part 17 may extend along a straight line which passes through the center of the cell culture vessel 10, as an example. Note, here, that the center of the cell culture vessel 10 refers to, but is not limited to, the center of gravity of the cell culture vessel 10, as an example. For example, the center may refer to an intersection of a plurality of diagonal lines out of diagonal lines connecting the vertices of the cell culture vessel 10 having a polyhedral shape.

The shape of the shaft part 17 is not limited in particular, and may have a rod-like shape, as an example (see Fig. 1). A material of the shaft part 17 may be a non-biocompatible material, provided that the material has a certain degree of strength.

The shaft part 17 may be connected to a rotating mechanism, such as a stepping motor 38, which is capable of rotating the cell culture vessel 10 on the shaft part 17 in a given direction at a given rotational speed. The direction of rotation, the rotational speed, and the duration of the rotation of the stepping motor 38 are controlled by a controller 39. By configuring, as in the present embodiment, the cell culture vessel 10 such that the cell culture vessel 10 is rotated on the shaft part 17 by a uniaxial rotating mechanism, it is possible to carry out an observation with use of a microscope 40 while rotating the cell culture vessel 10 on the shaft part 17. Note, however, that in the present embodiment, instead of the stepping motor 38, an observer may hold the shaft part 17 of the cell culture vessel 10 with use of tweezers or the like and manually rotate the cell culture vessel 10.

### (Wall part)

The cell culture vessel 10 may include a wall part (not illustrated) on at least some of the faces of the polyhedral shape. The wall part may cover the window part 12, or the wall part itself may have a role of the window part 12. The cell culture vessel 10 that includes the wall part easily retains the shape of a gel composition accommodated therein. The gel composition herein refers to the aqueous liquid after gelation. At least one of the faces of the polyhedral shape preferably does not include the wall part in order to inject thereinto the cell or cell tissue and/or the aqueous liquid.

Examples of a material of which the wall part is made include hydrogels such as agarose gel, polyacrylamide gel, sodium alginate, and collagen gel. The wall part is preferably light-transmissive in terms of, for example, ease of observation of the cell or cell tissue.

### (Aqueous liquid)

The cell or cell tissue is accommodated in the cell culture vessel 10 together with an aqueous liquid. The aqueous liquid is a liquid in which water is a main solvent (solvent in which water is contained in the largest volume). It is preferable that the water be contained in the solvent in an amount of not less than 50 vol%. Alternatively, it is sometimes preferable that the water be contained in the solvent in an amount of not less than 60 vol%, not less than 70 vol%, not less than 80 vol%, not less than 90 vol%, not less than 95 vol%, not less than 96 vol%, not less than 97 vol%, not more than 98 vol%, not more than 99 vol%, or 100 vol%. The aqueous liquid only needs to be a liquid capable of gelation. For example, the aqueous liquid may contain an extracellular matrix containing, for example, collagen, laminin, entactin, proteoglycan, fibrin, fibronectin, and fibrinogen. The aqueous liquid may contain, for example, TGF-β, a fibroblast cell growth factor, and/or a tissue plasminogen activating factor. The extracellular matrix or each of the factors can have a concentration that is selected as appropriate. Matrigel (registered trademark) may be used as the aqueous liquid.

The cell may be a cell tissue that has a structure assembled in a certain pattern, or may be a cell that does not have such a tissue structure. Alternatively, a cell tissue may be grown by culturing a cell that does not have a tissue structure.

The cell culture vessel 10 may accommodate two or more types of the aqueous liquid. In a case where the cell culture vessel 10 accommodates two or more types of the aqueous liquid, cells or cell tissues contained in the respective two or more types of the aqueous liquid may be identical to or different from each other. The aqueous liquid that does not include the cell or cell tissue may be accommodated in the cell culture vessel.

Even two or more types of the aqueous liquid that are accommodated in the cell culture vessel are each maintained in a corresponding compartment by surface tension. After the aqueous liquid is accommodated in the cell culture vessel 10, gelation of the aqueous liquid occurs. Gelation of the aqueous liquid does not mix gels between compartments. A cell culture vessel of an aspect of the present invention makes it possible to separate two or more types of the gel composition different from each other in composition. Separation of the two or more types of the gel composition different from each other in composition makes it possible to initiate three-dimensional culture with appropriate disposition (intended disposition) of the cell or cell tissue, or an extracellular matrix. Furthermore, use of the cell culture vessel 10 enables a worker to carry out, by an easy operation of injection of the aqueous liquid, organoid culture that is designed with a greatly increased degree of freedom. Moreover, separation of the two or more types of the gel composition different from each other in composition makes it possible to change morphology of the cell or cell tissue, or the extracellular matrix for each separation.

In order to promote gelation, the cell culture vessel 10 that is filled with a gel composition including the cell or cell tissue may be warmed to a desired temperature with use of, for example, an incubator. Examples of a treatment for promoting gelation include addition of ions to the aqueous liquid and UV irradiation to the aqueous liquid.

Culture may be carried out by placing, in a liquid culture medium, the cell culture vessel 10 that is filled with the gel composition including the cell or cell tissue.

After a certain period of culture, culture and/or the like may be further carried out by removing the cell culture vessel 10 that covers the gel composition including the cell or cell tissue.

The cell culture vessel 10 can be manufactured by, for example, a publicly-known technique such as a 3D printer.

### [Variation of cell culture vessel]

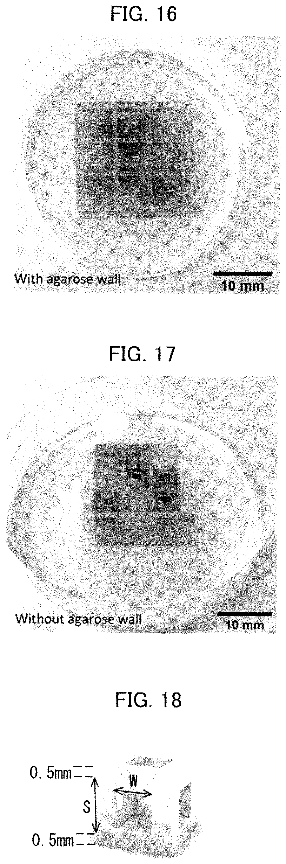

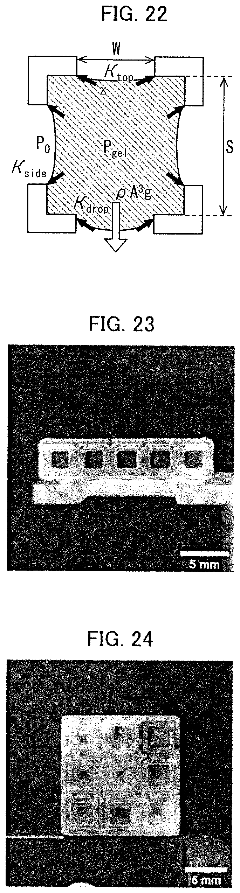

A variation of an individual compartment constituting the cell culture vessel is illustrated in, for example, Figs. 18 and 22. This compartment is a compartment having a cubic shape, and differs from the compartments having a cubic shape and illustrated in, for example, Figs. 1 and 2 in that a cube has sides (corresponding to a framework surrounding the compartment) having an L-shaped cross-section (see also Fig. 22). Note that the cross-section of the sides of the cube refers to a cross-section obtained by cutting the sides along a plane orthogonal to the sides.

The compartment illustrated in Fig. 18 has a cubic shape that is constituted by six square faces each having a single opening in a central part thereof. W in Fig. 18 is a length corresponding to each side of a square-shaped opening (a part in which the window part 12 (described later) is provided). S in Fig. 18 is an internal dimension of the compartment. Here, S is a length obtained by subtracting 1 mm from a length of each side (external dimension) of the compartment. In the compartment illustrated in Fig. 18 and the like, a relationship S>W is satisfied.

For example, from the point that an increase in area of contact between the aqueous liquid and the framework achieves higher surface tension and from the point of observation of, for example, imaging of the cell in the cell culture vessel, W is preferably 1 mm to 4.0 mm, more preferably 1.5 mm to 3.5 mm, and even more preferably 2.0 mm to 3.0 mm. Furthermore, S is preferably in a range of 1 mm to 10 mm, and more preferably in a range of 1 mm to 5 mm. Moreover, a difference (S-W) between S and W is preferably 0 to 6.0 mm, more preferably 0.5 mm to 5.5 mm, and even more preferably 1.0 mm to 5.0 mm.

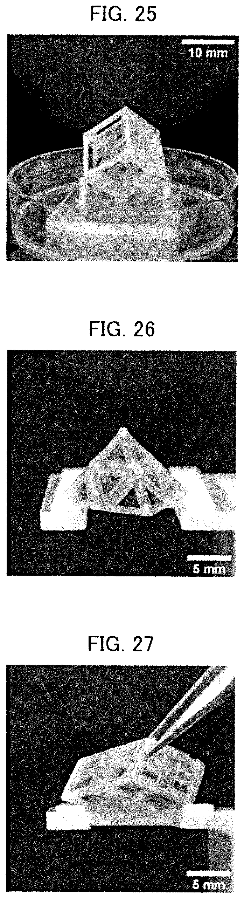

Note that the L-shaped cross-section is not limited to a case where an included angle is approximately 90 degrees (see Fig. 22). There may also be a case where the included angle is an acute angle or an obtuse angle (see, for example, Fig. 27). That is, the included angle formed by the L-shaped cross-section varies in accordance with the shape of the compartment.

Each of the six square faces constituting the compartment illustrated in Fig. 18 can be also understood as a square face that has an opening which is surrounded by edges having a given width. In the case of Fig. 18, the given · width of the edges is (S-W) × 1/2+0.5 mm assuming that S and W are dimensions in mm unit.

The cell culture vessel only needs to be constituted by a plurality of individual compartments illustrated in, for example, Figs. 18 and 22 and placed as in the case of the compartments illustrated in, for example, Figs. 1 and 2. Alternatively, a single cell culture vessel may be constituted as necessary by a combination of a compartment of a type illustrated in, for example, Figs. 18 and 22 and the compartments illustrated in, for example, Figs. 1 and 2.

### [2. Observation apparatus and microscope]

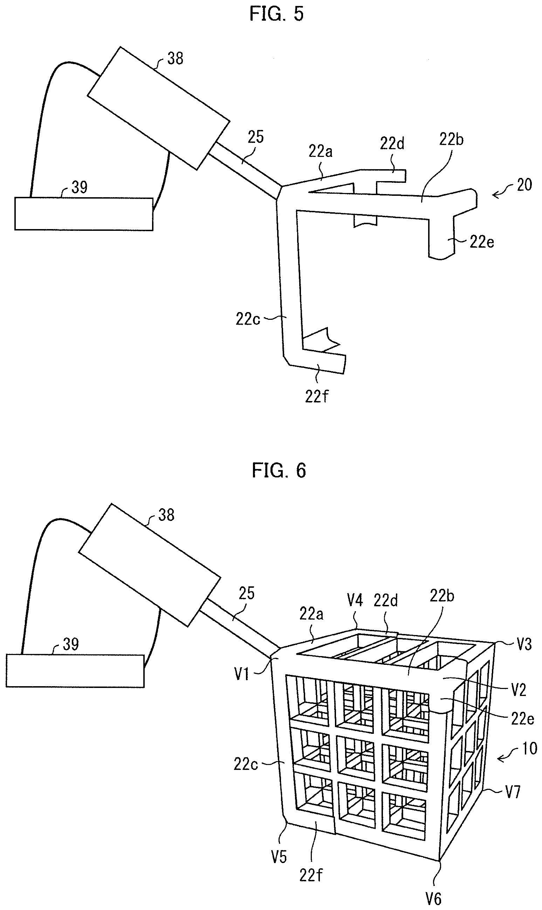

An example of an observation apparatus and a microscope in accordance with an aspect of the present invention will be described in detail with reference to Figs. 5 to 7.

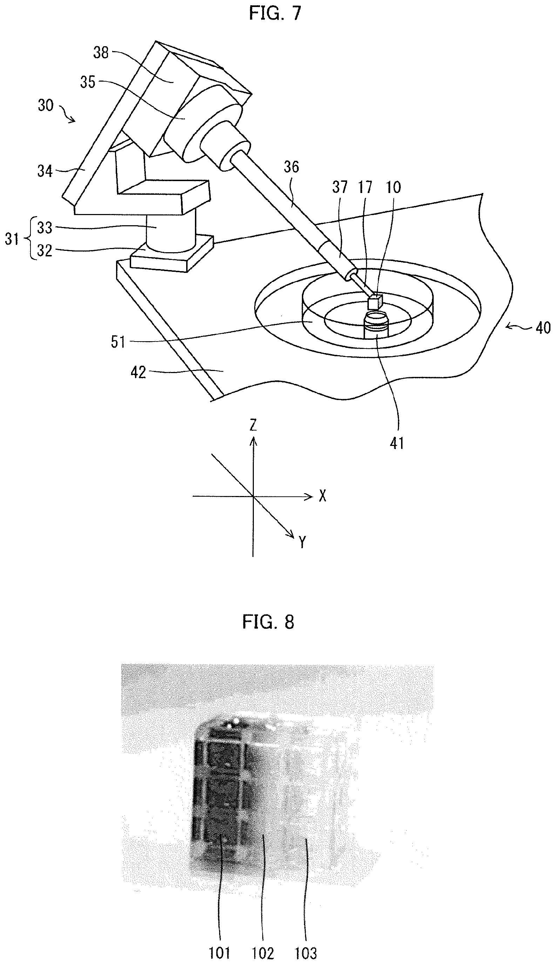

As illustrated in Fig. 7, an observation apparatus 30 includes (i) the cell culture vessel 10 which includes the shaft part 17 and (ii) a rotating mechanism (stepping motor 38). It is possible to obtain time-lapse images of the cell or cell tissue by mounting the observation apparatus 30 on a microscope 40.

Fig. 7 is a perspective view illustrating a state in which the observation apparatus 30 in accordance with Embodiment 1 of the present invention is mounted on the microscope 40. Coordinate axes illustrated in Fig. 7 are a coordinate system at rest which is expressed by X-, Y-, and Z-axes with the Z-axis as a vertical direction. The following description will discuss the observation apparatus 30 and the microscope 40 on which the observation apparatus 30 is mounted, in accordance with Embodiment 1 of the present invention, with reference to Fig. 7.

The observation apparatus 30 includes the cell culture vessel 10 and a rotating mechanism which holds the shaft part 17 of the cell culture vessel 10 and rotates the cell culture vessel 10 on the shaft part 17.

More specifically, as illustrated in Fig. 7, the observation apparatus 30 in accordance with Embodiment 1 includes a Z-axis mechanical stage 31, an obliquely fixed holder 34, a rotating stage 35, an extending part 36, a collet chuck 37, the stepping motor 38, and the cell culture vessel 10. Note, here, that the above rotating mechanism is constituted by, for example, the obliquely fixed holder 34, the extending part 36, the collet chuck 37, and the stepping motor 38.

The observation apparatus 30 in accordance with Embodiment 1 is mounted on a microscope stage 42 of the microscope 40.

The Z-axis mechanical stage 31 of the observation apparatus 30 includes (i) a base 32 which is mounted on the microscope stage 42 of the microscope 40 and (ii) a pedestal part 33 which extends vertically upward with respect to the base 32. The height of the pedestal part 33 in a Z-axis direction can be adjusted. This makes it possible to finely adjust, in the Z-axis direction, the position of the cell culture vessel 10 which is held by the collet chuck 37 (described later).

The obliquely fixed holder 34 is a plate-like member. The obliquely fixed holder 34 is connected to the pedestal part 33 of the Z-axis mechanical stage 31 at an angle with the pedestal part 33. The angle formed by the obliquely fixed holder 34 and the pedestal part 33 of the Z-axis mechanical stage 31 is set such that at least any of lines normal to the faces of the window part included in the cell culture vessel 10 can be parallel to the optical axis of a lens barrel 41. For example, in a case where the cell culture vessel 10 having a cubic shape is used, the obliquely fixed holder 34 is connected to an upper part of the pedestal part 33 of the Z-axis mechanical stage 31 at an angle of 135 degrees with respect to the pedestal part 33 of the Z-axis mechanical stage 31, as illustrated in Fig. 7. That is, in the embodiment illustrated in Fig. 7, the obliquely fixed holder 34 is fixed to the pedestal part 33 of the Z-axis mechanical stage 31 at an angle of 45 degrees with respect to a horizontal direction (X-Y direction).

To a lower surface of the obliquely fixed holder 34, the rotating stage 35 having a disk shape is provided. The rotating stage 35 is driven by the stepping motor 38 to rotate both clockwise and counterclockwise. By rotating the rotating stage 35, it is possible to rotate, both clockwise and counterclockwise, a member which is attached to an end of the rotating stage 35. Note that the rotating stage 35 may be configured to be rotated automatically with use of the stepping motor 38 or the like as described above or may be alternatively configured to be rotated manually by the observer.

The extending part 36 is connected to the center of the rotating stage 35. The extending part 36 extends linearly in a direction perpendicular to the obliquely fixed holder 34 and the rotating stage 35, i.e., in a direction at an angle of 45 degrees with respect to the horizontal direction (X-Y direction). The collet chuck 37 is connected to an end of the extending part 36. The collet chuck 37 holds the shaft part 17 of the cell culture vessel 10. For example, the collet chuck 37 to which the cell culture vessel 10 is attached may be held at an angle of 45 degrees with respect to a horizontal plane so as to hold the cell culture vessel 10 horizontally. As illustrated in Fig. 7, the cell culture vessel 10 is attached to the collet chuck 37 in a state in which the cell culture vessel 10 is immersed in the liquid culture medium in a dish (or well) 51.

Note that instead of the collet chuck 37 to which the cell culture vessel 10 is attached, a fixing tool 20 which is attached to the cell culture vessel 10 and which includes no shaft part 17 may be attached to the extending part 36.

Thus, by rotating the rotating stage 35, it is possible to rotate the cell culture vessel 10, attached to the end of the collet chuck 37, on the shaft part 17 at a given speed, while keeping the extending part 36 at an angle of 45 degrees with respect to the horizontal direction. The rotational speed and the duration of rotation of the rotating stage 35 (i.e., the rotational speed, the stopping time, and the duration of rotation of the cell culture vessel 10) can be set arbitrarily. This enables (i) continuous rotation and also (ii) an intermittent operation in which rotation and a stop are repeated. For example, the rotational speed may be set such that the cell culture vessel 10 is rotated clockwise twice per minute (i.e., rotated 120 degrees in 10 seconds), and the duration of the rotation may be set to three days, during which a cycle in which (i) after the cell culture vessel 10 has been rotated 120 degrees, the rotation of the cell culture vessel 10 is stopped for 110 seconds and then (ii) the cell culture vessel 10 is rotated again clockwise 120 degrees is repeated.

### <Observation of cell or cell tissue with microscope>

The following description will discuss a method of (i) setting, in the microscope 40, the cell culture vessel 10 in a state of being immersed in the liquid culture medium and being connected to the observation apparatus 30 and (ii) carrying out an observation. As the microscope 40, a microscope conventionally used can be used. For example, a confocal laser scanning microscope or a two-photon microscope can be used as the microscope 40. Note that Fig. 7 illustrates an inverted microscope as an example, but the microscope 40 may be an upright microscope.

As illustrated in Fig. 7, the cell culture vessel 10 is immersed in the liquid culture medium in the dish (or well) 51.

In a case where the cell culture vessel 10 is observed, the cell culture vessel 10 in a state of being immersed in the dish 51 is placed on the microscope stage 42 so that the cell or cell tissue accommodated in the cell culture vessel 10 is located within the field of view of the lens barrel 41 of the microscope 40.

The Z-axis mechanical stage 31 of the observation apparatus 30 is then mounted at a given position on the microscope stage 42 of the microscope 40. Subsequently, the cell culture vessel 10 in the state of being horizontally immersed in the dish 51 is connected to the end of the extending part 36 of the observation apparatus 30 via the collet chuck 37. Thereafter, the position in the Z-axis direction at which the cell culture vessel 10 is set is finely adjusted with use of the Z-axis mechanical stage 31.

When the position of the cell culture vessel 10 is determined, the rotational speed, the stopping time in the intermittent operation, and the duration of the rotation of the rotating stage 35 are set. Observing the cell or cell tissue (i.e., obtaining images) while the rotation is being stopped is repeated.

The cell culture vessel 10 which includes the shaft part 17 was described above. However, the cell culture vessel 10 which includes no shaft part 17 may be used to observe the cell or cell tissue.

The following description will discuss the fixing tool 20 used to fix the cell culture vessel 10 which includes no shaft part 17. The cell culture vessel 10 has a configuration similar to that of the above-described cell culture vessel 10, except that the cell culture vessel 10 includes no shaft part 17. Fig. 5 illustrates an example of the fixing tool 20 used to fix the cell culture vessel 10 which includes no shaft part 17. Fig. 6 illustrates an example of a state in which the fixing tool 20 is fitted on the cell culture vessel 10.

### (Fixing tool)

As illustrated in Fig. 5, the fixing tool 20 includes a holding part 22 which holds the cell culture vessel 10 and a shaft part 25 which is connected to the holding part 22.

The holding part 22 includes three holding portions 22a, 22b, and 22c which extend in respective directions that form right angles with each other and which have respective substantially equal lengths. The three holding portions 22a, 22b, and 22c have, at ends thereof, claws 22d, 22e, and 22f, respectively.

In a state in which the fixing tool 20 is fitted on the cell culture vessel 10, the holding portions 22a, 22b, and 22c are disposed along respective portions of the outer framework 11 which are provided at positions that correspond to respective three sides forming the vertex V1 of the cell culture vessel 10 (a side connecting V1 and V4, a side connecting V1 and V2, and a side connecting V1 and V5), as illustrated in Fig. 6. The claws 22d, 22e, and 22f hold the vertices V4, V2, and V5, respectively, of the framework adjacent to the vertex V1 of the cell culture vessel 10.

In a state in which the fixing tool 20 is fixed to the cell culture vessel 10, the shaft part 25 of the fixing tool 20 extends, from the vertex V1 of a polyhedral shape (cubic shape) of the cell culture vessel 10, outward in a direction that is not parallel to any of lines normal to faces of the polyhedral shape.

When the cell or cell tissue accommodated in the cell culture vessel 10 is observed, the cell culture vessel 10 is rotated on the shaft part 25 of the fixing tool 20. As a result, it is possible to obtain images of the cell or cell tissue which have high resolution in the Z-axis direction, as in the case of the cell culture vessel 10 which includes the shaft part 17.

### [3. Culture method]

A culture method of an aspect of the present invention is a method of culturing the cell or cell tissue accommodated in the above cell culture vessel. The culture method of an aspect of the present invention includes the step of accommodating, in the compartments 100 of the cell culture vessel 10, the gel composition including the cell or cell tissue (hereinafter may be abbreviated to an "accommodation step").

### (Accommodation step)

In the accommodation step, for example, the aqueous liquid including the cell or cell tissue is accommodated in the compartments 100 of the cell culture vessel 10, and the gel composition including the cell or cell tissue is accommodated in the compartments 100 by gelation of the aqueous liquid. The aqueous liquid that includes no cell or cell tissue may be accommodated in the compartments 100 of the cell culture vessel 10.

### (Another step)

The culture method of an aspect of the present invention may include another step different from the accommodation step. Examples of the another step include a wall part forming step.

### (Wall part forming step)

In the wall part forming step, the wall part is formed on at least some of the faces of the polyhedral shape of the cell culture vessel 10. For example, as shown in Examples, the wall part can be formed in a case where the cell culture vessel 10 is placed on a material in liquid form of which the wall part is made and which is heated to a certain temperature, and the material in liquid form of which the wall part is made is subjected to gelation. The wall part forming step is preferably carried out before the accommodation step.

### [4. Observation method]

An observation method of an aspect of the present invention is a method of observing the cell or cell tissue accommodated in the above cell culture vessel. The observation method of an aspect of the present invention includes, in a case where the cell culture vessel 10 includes the shaft part 17, the step of rotating the cell culture vessel 10 on the shaft part 17 (hereinafter may be abbreviated to a "first rotation step"). The observation method of an aspect of the present invention includes, in a case where the cell culture vessel 10 includes no shaft part, the step of using the fixing tool 20 to hold the cell culture vessel 10 and rotate the cell culture vessel 10 on the shaft part of the fixing tool (hereinafter may be abbreviated to a "second rotation step").

### [5. Summary of embodiments]

The embodiments of the present invention include, for example, the following aspects.
<1> A cell culture vessel including a plurality of compartments each having a corresponding three-dimensional shape and each configured to be able to retain therein an aqueous liquid by surface tension, wherein any adjacent compartments out of the plurality of compartments communicate with each other.
<2> A cell culture vessel including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other.
<3> The cell culture vessel recited in <1> or <2>, wherein the cell culture vessel has an external form having a polyhedral shape, the cell culture vessel further including a window part which is light-transmissive and which is provided so as to be located at positions that correspond to a respective plurality of faces out of faces of the polyhedral shape.
<4> The cell culture vessel recited in any one of <1> through <3>, wherein the cell culture vessel has an external form having a polyhedral shape, the cell culture vessel further including a shaft part which extends, from any of vertices of the polyhedral shape, outward in a direction that is not parallel to any of lines normal to faces of the polyhedral shape.
<5> The cell culture vessel recited in any one of <1> through <4>, wherein the plurality of compartments are placed side by side in at least two directions selected from an X direction, a Y direction, and a Z direction.
<6> The cell culture vessel recited in any one of <1> through <5>, wherein the plurality of compartments are surrounded by a framework corresponding to sides of a hexahedron.
<7> The cell culture vessel recited in any one of <1> through <6>, wherein the framework corresponding to the sides of the polyhedron has an L-shaped cross-section.
<8> The cell culture vessel recited in any one of <1> through <7>, wherein at least some of the plurality of compartments are filled with a gel composition including a cell or cell tissue.
<9> The cell culture vessel recited in <8>, wherein the at least some of the plurality of compartments are filled with two or more types of the gel composition.
<10> An observation apparatus including: a cell culture vessel recited in any one of <1> through <9>, the cell culture vessel having an external form having a polyhedral shape; a fixing tool for fixing the cell culture vessel; and a rotating mechanism which rotates the cell culture vessel, the fixing tool including: a holding part which holds the cell culture vessel; and a shaft part which is connected to the holding part and which extends, from any of vertices of the polyhedral shape, outward in a direction that is not parallel to any of lines normal to faces of the polyhedral shape, the rotating mechanism holding the shaft part and rotating, on the shaft part, the cell culture vessel held by the fixing tool.
<11> An observation apparatus including a cell culture vessel recited in <4>, the cell culture vessel having an external form having a polyhedral shape, the observation apparatus holding the shaft part of the cell culture vessel and rotating the cell culture vessel on the shaft part.
<12> A microscope including an observation apparatus recited in <10> or <11>.
<13> A method of culturing a cell or cell tissue accommodated in a cell culture vessel that has an external form having a polyhedral shape, the cell culture vessel including a plurality of compartments each having a corresponding three-dimensional shape and each configured to be able to retain therein an aqueous liquid by surface tension, wherein any adjacent compartments out of the plurality of compartments communicate with each other, or including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other, the method including accommodating, in the plurality of compartments, a gel composition including a cell or cell tissue.
<14> A method of observing a cell or cell tissue accommodated in a cell culture vessel that has an external form having a polyhedral shape, the cell culture vessel including a plurality of compartments each having a corresponding three-dimensional shape and each configured to be able to retain therein an aqueous liquid by surface tension, wherein any adjacent compartments out of the plurality of compartments communicate with each other, or including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other, the cell culture vessel further including a shaft part which extends, from any of vertices of the polyhedral shape, outward in a direction that is not parallel to any of lines normal to faces of the polyhedral shape, the method including rotating the cell culture vessel on the shaft part.
<15> A method of observing a cell or cell tissue accommodated in a cell culture vessel that has an external form having a polyhedral shape, the cell culture vessel including a plurality of compartments each having a corresponding three-dimensional shape and each configured to be able to retain therein an aqueous liquid by surface tension, wherein any adjacent compartments out of the plurality of compartments communicate with each other, or including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other, a fixing tool for fixing the cell culture vessel including: a holding part which holds the cell culture vessel; and a shaft part which is connected to the holding part and which extends, from any of vertices of the polyhedral shape, outward in a direction that is not parallel to any of lines normal to faces of the polyhedral shape, the method including using the fixing tool to hold the cell culture vessel and rotating the cell culture vessel on the shaft part.

### Examples

In the following examples, % refers to mass% unless otherwise stated.

### [Evaluation Example 1] Preparation of cell culture vessel and evaluation by accommodation of three types of gel composition

The cell culture vessel 10 (1 cm per side) illustrated in Fig. 1 was prepared. The cell culture vessel 10 was prepared by a 3D printer (manufactured by KEYENCE CORPORATION). The inner part of the cell culture vessel 10 is equally divided into 27 compartments as illustrated in Fig. 1. The compartments thus obtained have a substantially cubic shape and communicate with each other as illustrated in Fig. 1.

A pipette was used to fill nine compartments including the compartment 101 with Matrigel (registered trademark) mixed with an edible blue pigment. The pipette was used to fill nine compartments including the compartment 102 with Matrigel (registered trademark) mixed with fluorescence beads. The pipette was used to fill nine compartments including the compartment 103 with Matrigel (registered trademark).

After all the compartments were filled with gels, a frame (frame parts) filled with gels was put into and stored in an incubator at 37°C for approximately 25 minutes before start of mixing of heterogeneous gels due to diffusion of the gels having adhered to each other.

Fig. 8 illustrates the cell culture vessel after storage in the incubator. It has been found that three types of a gel composition were separated from each other.

### [Evaluation Example 2] Preparation of cell culture vessel and evaluation by accommodation of two types of gel composition

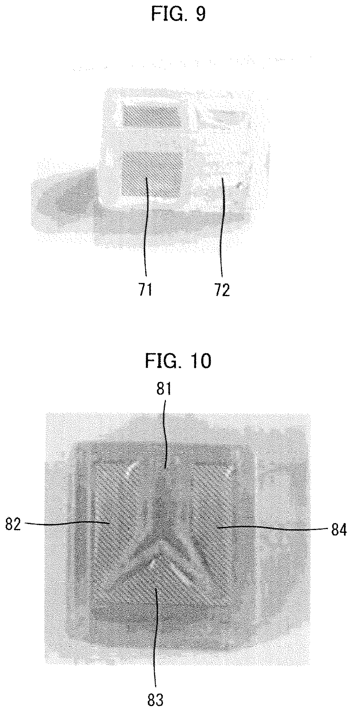

A cell culture vessel 70 (4 mm in length × 8 mm in width × 4 mm in height) having a rectangular parallelepiped shape and having two compartments of the same size was prepared as illustrated in Fig. 9. A frame was prepared by a 3D printer. The compartments have a substantially cubic shape and communicate with each other.

A pipette was used to fill one (a compartment 71 on the left side in Fig. 9) of the two compartments with Matrigel (registered trademark) mixed with an edible blue pigment. The pipette was used to fill the other (a compartment 72 on the right side in Fig. 9) of the two compartments with Matrigel (registered trademark) mixed with an edible yellow pigment.

After all the compartments were filled with a gel composition, the frame filled with gels was put into and stored in an incubator at 37°C for approximately 25 minutes before start of mixing of heterogeneous gels due to diffusion of the gels having adhered to each other.

Fig. 9 illustrates the cell culture vessel after storage in the incubator. It has been found that two types of the gel composition were separated from each other.

### [Evaluation Example 3] Preparation of cell culture vessel and evaluation by accommodation of two types of gel composition

A cell culture vessel (1 cm in length × 1 cm in width × 0.5 cm in height) having a cubic shape and having four compartments was prepared as illustrated in Fig. 10. A frame was prepared by a 3D printer. The compartments communicate with each other. Note that frames prepared in the following Evaluation Examples 4 to 6 were each prepared by a 3D printer.

A pipette was used to fill a compartment 81 illustrated in Fig. 10 with Matrigel (registered trademark) mixed with an edible blue pigment. The pipette was used to fill a compartment 82 with Matrigel (registered trademark) mixed with an edible red pigment. The pipette was used to fill a compartment 83 with Matrigel (registered trademark) mixed with the edible red pigment. The pipette was used to fill a compartment 84 with Matrigel (registered trademark) mixed with the edible red pigment.

After all the compartments were filled with a gel composition, the frame filled with gels was put into and stored in an incubator at 37°C for approximately 25 minutes before start of mixing of heterogeneous gels due to diffusion of the gels having adhered to each other.

Fig. 10 illustrates the cell culture vessel after storage in the incubator. It has been found that two types of the gel composition were separated from each other.

### [Example 1] Culture of cell with cell culture vessel

### (1) Preparation of wall part (outer wall)

Onto slide glass, approximately 20 µL to 200 µL of 1.5% agarose was dropped that was in liquid form and that had been warmed to 85°C. Next, the cell culture vessel prepared in Evaluation Example 1 was placed on top of the agarose, and the slide glass as a whole was cooled so that the agarose gelated.

After gelation of the agarose, the agarose protruding from the frame was cut off and removed. Thus, an agarose outer wall was prepared for the frame. The agarose outer wall was prepared on five out of six faces of the prepared frame.

### (2) Filling of the HUVEC-containing gel

The compartments 101 and 103 illustrated in Fig. 1 were filled with 50 µL of a human umbilical vein endothelial cell (HUVEC)-containing gel. As a gel, Matrigel (registered trademark) containing 40% collagen was used. A pipette was used to fill the HUVEC-containing gel from the face of the frame on which face no agarose outer wall was prepared.

### (3) Filling of HepG2-HFL-containing gel

The compartment 102 illustrated in Fig. 1 was filled with 50 µL of a HepG2-HFL spheroid-containing gel. HepG2-HFL spheroid is spheroid composed of a human hepatoma-derived cell (mHepG2) and a human fibroblast (HFL). As a gel, Matrigel (registered trademark) containing 42.4% collagen and 15.2% fibrin was used. The pipette was used to fill the HepG2-HFL spheroid-containing gel from the face of the frame on which face no agarose outer wall was prepared.

### (4) Gel crosslinking and cell culture

After an internal space of the frame was filled with gels, the frame filled with the gels was put into and stored in an incubator at 37°C for approximately 25 minutes before start of mixing of heterogeneous gels due to diffusion of the gels having adhered to each other. After it was confirmed that the gels had been crosslinked, the frame was placed in a liquid culture medium, and cell culture was carried out in the incubator at 37°C for 8 days.

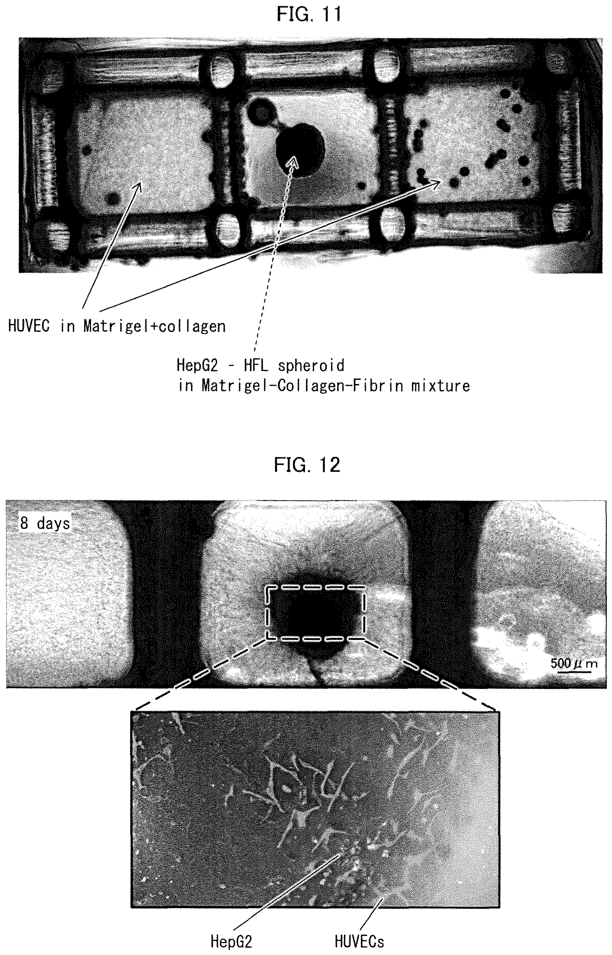

Fig. 11 is an enlarged photograph of the frame after 0-day culture. Fig. 12 is an enlarged photograph of the frame after 8-day culture. As illustrated in Figs. 11 and 12, it has been found that use of the cell culture vessel 10 prepared in Evaluation Example 1 to change compositions of an aqueous liquid in a central part of the frame and an aqueous liquid on both sides of the frame makes it possible to separate heterogeneous extracellular matrices in accordance with a difference in surface tension between the aqueous liquids. It has also been found that heterogeneous cells can be separated in a case where cells or cell tissues to be cultured are changed in the central part of the frame and on both sides of the frame.

In Example 1, an extracellular matrix was contained in a gel. However, it is considered that filling the gel with a cell in place of the extracellular matrix makes it possible to control an initial position or the shape of a cell.

### [Evaluation Example 4] Study of frame shape

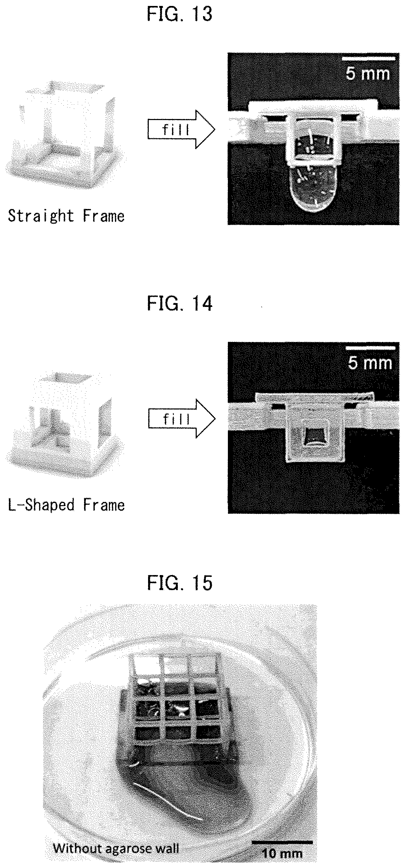

Fig. 13 illustrates a frame (5 mm per side) of a cell culture vessel, the frame having a substantially quadrangular vertical cross-section. The frame illustrated in Fig. 13 may be hereinafter referred to as a straight frame. Fig. 14 illustrates a frame (5 mm per side) of a cell culture vessel, the frame having a substantially L-shaped vertical cross-section. The frame illustrated in Fig. 14 may be hereinafter referred to as an L-shaped frame.

The frame illustrated in Fig. 13 was filled with Matrigel containing 30% collagen (hereinafter referred to as a "collagen gel 1") without the agarose outer wall prepared for the frame, and was put into and stored in an incubator at 37°C for approximately 25 minutes. After the frame was stored in the incubator, in a case where the cell culture vessel prepared was lifted with the frame filled with the collagen gel 1, the collagen gel 1 sometimes dropped out of the frame depending on viscosity of the agarose outer wall, as illustrated on the right in Fig. 13. In contrast, after the frame illustrated in Fig. 14 was filled with the collagen gel 1 without the agarose outer wall prepared for the frame and was stored in the incubator at 37°C for approximately 25 minutes, in a case where the cell culture vessel prepared was lifted with the frame filled with the collagen gel 1, the collagen gel 1 did not drop out of the frame, as illustrated on the right in Fig. 14. It is suggested that this is because an increase in area of contact between the gel and the frame due to the L-shaped frame resulted in an increase in surface tension.

Figs. 15 and 17 show results of filling the collagen gel 1 into the frame of the cell culture vessel for which frame no agarose outer wall was prepared. Fig. 16 shows a result of filling the collagen gel 1 into the frame of the cell culture vessel for which frame the agarose outer wall was prepared. In Figs. 15 and 16, a straight frame having nine compartments (5 mm per side of each of the compartments) having the same size and having a substantially cubic shape was used. In Fig. 17, an L-shaped frame having nine compartments (5 mm per side of each of the compartments) having the same size and having a substantially cubic shape was used. In Figs. 15 to 17, the compartments were filled with three types of the collagen gel 1 (collagen gel 1 mixed with respective edible red, blue, and yellow pigments).

In the case of the straight frame for which no agarose outer wall was prepared, it was impossible to maintain the collagen gel 1 in the compartments, as illustrated in Figs. 15 and 16. As illustrated in Fig. 16, it was possible to maintain the collagen gel 1 in the compartments. However, it was observed after storage in the incubator at 37°C for approximately 25 minutes that the collagen gel 1 of three colors was mixed. This prevented the collagen gel 1 of each of the colors from being localized in a corresponding desired compartment. This is considered to have been caused by being greatly influenced by gravity. In contrast, as illustrated in Fig. 17, use of the L-shaped frame allowed the collagen gel 1 of each of the colors to be localized in a corresponding desired compartment.

### [Evaluation Example 5] Study of condition for L-shaped frame

Next, a physical force preventing an influence of gravity was evaluated for an L-shaped frame. An L-shaped frame illustrated in Fig. 18 and having a substantially cubic shape was prepared. A frame width was fixed to 1 mm per side, and frames in which W in Fig. 18 was 2.0 mm to 4.0 mm and S in Fig. 18 was 3.0 mm to 7.0 mm were prepared.

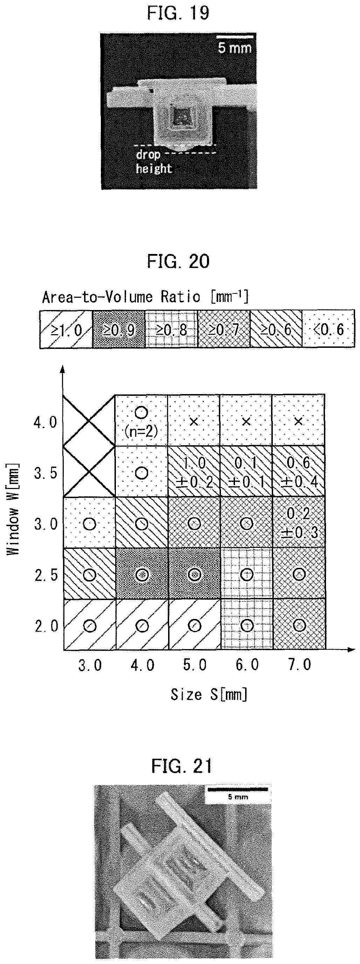

The L-shaped frame illustrated in Fig. 18 was filled with the collagen gel 1, and the cell culture vessel prepared was lifted with the frame filled with the collagen gel 1, as illustrated in Fig. 19, so that the size of a droplet formed in a bottom part of the cell culture vessel (the height of the droplet from the bottom part of the cell culture vessel) was measured. Fig. 20 shows a measurement result. A frame in which W=4.0 mm and S=4.0 mm was subjected to experiments twice (n=2), and the other frames were subjected to experiments three times (n=3).

In Fig. 20, the vertical axis shows the length of W in Fig. 18, and the horizontal axis represents the length of S in Fig. 18. "∘" in Fig. 20 shows that no droplet was observed. "##" shows that a droplet was observed. _{"}×_{"} shows that a droplet dropped and it was impossible to measure the height of the droplet.

As illustrated in Fig. 20, it has been found that a droplet is easily generated (the collagen gel 1 easily drops out of the frame) in a case where both W and S are beyond respective certain ranges.

Figs. 21 and 22 show results of evaluations of a curve on an interface between a gel composition and air. It has been found that the L-shaped frame should be designed in consideration of the following points: the initial shape of the gel composition filled into the L-shaped frame (initial hydrogel shape from pipette); and the shape of the gel composition at equilibrium (hydrogel shape at equilibrium). These evaluations suggest that an interaction between physical forces prevents an influence of the weight in the L-shaped frame and that Laplace pressure is important in the L-shaped frame.

### [Evaluation Example 6] Preparation of cell culture vessel and evaluation by accommodation of three types of gel composition

An L-shaped frame was used to prepare cell culture vessels having various shapes. Compartments of the frame were filled with respective three types of the collagen gel 1 (collagen gel 1 mixed with respective edible red, blue, and yellow pigments), and the frame was stored in an incubator at 30°C for approximately 25 minutes. Figs. 23 to 28 illustrate the cell culture vessels after storage in the incubator. Compartments of the cell culture vessels communicate with each other as illustrated in Fig. 1.

Fig. 23 illustrates a cell culture vessel prepared with use of a frame which has five compartments and in which S=4.0 mm and W=3.0 mm. Fig. 24 illustrates a cell culture vessel prepared with use of a frame which has nine compartments and in which S=4.0 mm and W=2.5 mm. Each of the compartments of the frames illustrated in Figs. 23 and 24 has a substantially cubic shape.

Fig. 25 illustrates a cell culture vessel prepared with use of a frame which has 27 compartments and in which S=3.0 mm and W=2.0 mm. The cell culture vessel illustrated in Fig. 25 is further tilted along a diagonal axis at a desired angle by a fixing tool having a substantially cubic shape. Each of the compartments of the frame illustrated in Fig. 25 has a substantially cubic shape. As illustrated in Fig. 25, by tilting the cell culture vessel along the diagonal axis so that gravity is pulled toward an edge or a corner of the cell culture vessel, it was possible to prevent generation of a droplet of a desired aqueous liquid and prevent easy mixing of the desired aqueous liquid with an adjacent aqueous liquid.

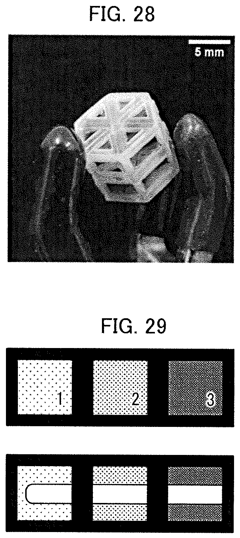

Fig. 26 illustrates a cell culture vessel prepared with use of a frame that has a substantially square pyramid shape and that has compartments having a substantially triangular pyramid shape and having 5 mm per side. Fig. 27 illustrates a cell culture vessel prepared with use of a frame (5 mm in length x 3 mm in width x 2.5 mm in height) that has a rectangular parallelepiped shape and that has eight compartments having the same size. Fig. 28 illustrates a cell culture vessel prepared with use of a frame (3 mm per side of a bottom face, 6 mm in height) that has a hexagonal prism shape and that has 12 compartments having a substantially triangular prism shape and having the same size.

### [Example 2] Formation of bronchus with use of cell culture vessel

Three L-shaped frames in which S=4.0 mm and W=3.0 mm were used to prepare cell culture vessels. Among three compartments illustrated in Fig. 29, the left compartment (compartment 1) was filled with Matrigel (registered trademark) only. The middle compartment (compartment 2) was filled with Matrigel (registered trademark) containing 0.15 mM Genipin (a crosslinking agent). The right compartment (compartment 3) was filled with Matrigel (registered trademark) containing 0.3 mM Genipin. The compartment 3 was also filled with human bronchial epithelial cells (normal human bronchial epithelial cells (NHBEs)) or mouse endothelial cells.

After filling, a frame filled with gels into which cells had been injected was put into and stored in an incubator at 37°C for approximately 25 minutes before start of mixing of heterogeneous gels due to diffusion of the gels having adhered to each other. After it was confirmed that the gels had been crosslinked, the frame was placed in a liquid culture medium, and culture was carried out for 96 hours (with respect to human lung cells) or 92 hours (with respect to mouse endothelial cells).

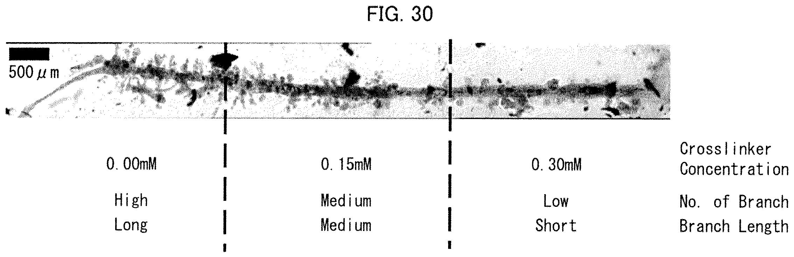

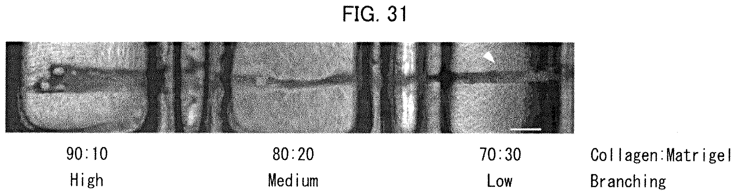

Fig. 30 shows a result of culturing the human bronchial epithelial cells. Fig. 31 shows a result of culturing the mouse endothelial cells. As is clear from Figs. 30 and 31, bronchi formed by culture were observed in the left compartment and in the middle compartment. Most bronchial branches were observed in the left compartment to which no Genipin had been added. A higher concentration of Genipin, which is the crosslinking agent, resulted in fewer bronchial branches. In the left compartment to which no Genipin had been added, the bronchial branches also had the longest length. A higher concentration of Genipin resulted in shorter bronchial branches. It has been found from these results that in tissue culture, tissue morphology can be changed by changing viscosity of an aqueous liquid with which each compartment of a cell culture device is filled.

It has been found from the above that use of any of the cell culture vessels (frames) prepared in Examples of the present invention makes it possible to initiate three-dimensional culture with appropriate disposition of an appropriate cell and an appropriate extracellular matrix, and enables organoid culture to be designed with a greatly increased degree of freedom. It has also been found that use of any of the frames prepared in Examples of the present invention makes it possible to carry out three-dimensional culture by a simple and easy operation of filling a frame with a gel including a cell or cell tissue, and requires no special apparatus or technique.

### Industrial Applicability

The present invention, which makes it possible to provide a system for screening a therapeutic agent with high therapeutic efficacy, can be used for, for example, a medical purpose.

### Reference Signs List

- 10, 70, 80: Cell culture vessel
- 11: Outer framework
- 12: Window part
- 15: Protruding part
- 16: End portion
- 17: Shaft part (of cell culture vessel)
- 18: Bearing
- 20: Fixing tool
- 22: Holding part
- 25: Shaft part (of fixing tool)
- 30: Observation apparatus
- 31: Z-axis mechanical stage
- 32: Base
- 33: Pedestal part
- 34: Obliquely fixed holder
- 35: Rotation stage
- 36: Extending part
- 37: Collet chuck
- 38: Stepping motor
- 39: Controller
- 41: Lens barrel
- 42: Microscope stage
- 51: Dish
- 60: Light-sheet microscope
- 61: Mirror
- 62: Light sheet
- 63: Laser emitting section
- 71, 72, 81-84, 100-104, 200: Compartment

## Claims

1. A cell culture vessel comprising
a plurality of compartments each having a corresponding three-dimensional shape and each configured to be able to retain therein an aqueous liquid by surface tension, wherein
any adjacent compartments out of the plurality of compartments communicate with each other.

2. A cell culture vessel comprising
a plurality of compartments surrounded by a framework corresponding to sides of a polyhedron,
the plurality of compartments each being configured to be able to retain therein an aqueous liquid by surface tension, wherein
any adjacent compartments out of the plurality of compartments communicate with each other.

3. The cell culture vessel as set forth in claim 1 or 2, wherein the cell culture vessel has an external form having a polyhedral shape,
said cell culture vessel further comprising
a window part which is light-transmissive and which is provided so as to be located at positions that correspond to a respective plurality of faces out of faces of the polyhedral shape.

4. The cell culture vessel as set forth in any one of claims 1 through 3, wherein the cell culture vessel has an external form having a polyhedral shape,
said cell culture vessel further comprising
a shaft part which extends, from any of vertices of the polyhedral shape, outward in a direction that is not parallel to any of lines normal to faces of the polyhedral shape.

5. The cell culture vessel as set forth in any one of claims 1 through 4, wherein the plurality of compartments are placed side by side in at least two directions selected from an X direction, a Y direction, and a Z direction.

6. The cell culture vessel as set forth in any one of claims 1 through 5, wherein the plurality of compartments are surrounded by a framework corresponding to sides of a hexahedron.

7. The cell culture vessel as set forth in any one of claims 2 through 6, wherein the framework corresponding to the sides of the polyhedron has an L-shaped cross-section.

8. The cell culture vessel as set forth in any one of claims 1 through 7, wherein at least some of the plurality of compartments are filled with a gel composition including a cell or cell tissue.

9. The cell culture vessel as set forth in claim 8, wherein the at least some of the plurality of compartments are filled with two or more types of the gel composition.

10. An observation apparatus comprising:
a cell culture vessel recited in any one of claims 1 through 9, the cell culture vessel having an external form having a polyhedral shape;
a fixing tool for fixing the cell culture vessel; and
a rotating mechanism which rotates the cell culture vessel,
the fixing tool including:
a holding part which holds the cell culture vessel; and
a shaft part which is connected to the holding part and which extends, from any of vertices of the polyhedral shape, outward in a direction that is not parallel to any of lines normal to faces of the polyhedral shape,
the rotating mechanism holding the shaft part and rotating, on the shaft part, the cell culture vessel held by the fixing tool.

11. An observation apparatus comprising
a cell culture vessel recited in claim 4, the cell culture vessel having an external form having a polyhedral shape,
the observation apparatus holding the shaft part of the cell culture vessel and rotating the cell culture vessel on the shaft part.

12. A microscope comprising an observation apparatus recited in claim 10 or 11.

13. A method of culturing a cell or cell tissue accommodated in a cell culture vessel that has an external form having a polyhedral shape,
the cell culture vessel
including a plurality of compartments each having a corresponding three-dimensional shape and each configured to be able to retain therein an aqueous liquid by surface tension, wherein any adjacent compartments out of the plurality of compartments communicate with each other, or
including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other,
said method comprising
accommodating, in the plurality of compartments, a gel composition including a cell or cell tissue.

14. A method of observing a cell or cell tissue accommodated in a cell culture vessel that has an external form having a polyhedral shape,
the cell culture vessel
including a plurality of compartments each having a corresponding three-dimensional shape and each configured to be able to retain therein an aqueous liquid by surface tension, wherein any adjacent compartments out of the plurality of compartments communicate with each other, or
including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other,
the cell culture vessel further including
a shaft part which extends, from any of vertices of the polyhedral shape, outward in a direction that is not parallel to any of lines normal to faces of the polyhedral shape,
said method comprising
rotating the cell culture vessel on the shaft part.

15. A method of observing a cell or cell tissue accommodated in a cell culture vessel that has an external form having a polyhedral shape,
the cell culture vessel
including a plurality of compartments each having a corresponding three-dimensional shape and each configured to be able to retain therein an aqueous liquid by surface tension, wherein any adjacent compartments out of the plurality of compartments communicate with each other, or
including a plurality of compartments surrounded by a framework corresponding to sides of a polyhedron, the plurality of compartments each being configured to be able to retain therein an aqueous liquid by surface tension, wherein any adjacent compartments out of the plurality of compartments communicate with each other,
a fixing tool for fixing the cell culture vessel including:
a holding part which holds the cell culture vessel; and
a shaft part which is connected to the holding part and which extends, from any of vertices of the polyhedral shape, outward in a direction that is not parallel to any of lines normal to faces of the polyhedral shape,
said method comprising
using the fixing tool to hold the cell culture vessel and rotating the cell culture vessel on the shaft part.
